# EUROPEAN PATENT APPLICATION

(11) **EP 3 527 256 A1**
(43) Date of publication of application: **21.08.2019**
(21) Application number: 17872988.5
(22) Date of filing: 23.11.2017
(51) Int. Cl.: A61N 1/18

(54) **FOOT DEVICE FOR TREATING VASCULAR COMPLAINTS IN THE LOWER LIMBS**

(30) Priority: 23.11.2016 ES 201631505
(71) Applicant: Sanchez Jaime, Maria del Pilar, 08970 Barcelona (ES)
(72) Inventor: Sanchez Jaime, Maria del Pilar, 08970 Barcelona (ES)
(74) Representative: Isern Patentes y Marcas S.L.
(86) International application number: PCT/ES2017/070775
(87) International publication number: WO 2018/096202

(57) **Abstract**

The invention relates to a foot device for treating vascular complaints in the lower limbs, which comprises an L-shaped support structure (2) with a soft inner covering (3) into which radio-frequency electrode plates (4, 4') are integrated, as an adjustable base for contact between the lower part of the leg and foot of the patient and the electrode plates (4, 4'), the structure (2) having securing means (6) for autonomously keeping the structure attached to the limb and keeping the electrode plates (4, 4') in contact with the patient's skin. The support structure (2) is rigid and comprises a vertical branch (2a) sized to accommodate and act as a support to the area of the rear end part of the patient's leg, and a horizontal branch (2b) sized to accommodate the sole of the patient's foot.

## Description

### OBJECT OF THE INVENTION

The invention, as expressed by the title of the present specification, relates to a foot device for treating vascular complaints in the lower limbs which provides structural and constituent characteristics that will be described in detail below and which represent a notable novelty in the current state of the art.

More specifically, the object of the invention is a device, the aim of which is to improve the application of energy treatments in the lower limbs, in particular by means of the application of radio-frequency waves in patients who have vascular complaints, such as diabetic foot, and which has a structural configuration that is specifically designed to provide an autonomous fastening with perfect ergonomics in the interaction of the electrodes on the skin of said limbs in a quick, practical and simple way, thereby avoiding constant monitoring by professionals who apply the treatment, further having an additional integrated circuit which, by means of the use of a conductive cream, provides it with the electroporation capability to introduce molecules of active medication in order to heal and repair ulcers and cutaneous lesions resulting from the circulatory and vascular complaint of the diabetic foot.

### FIELD OF APPLICATION OF THE INVENTION

The field of application of the present invention falls within the sector of the industry dedicated to the manufacturing of accessory devices for equipment and devices for medical treatment, with a particular focus on the field of equipment and devices that can be applied for vascular complaints in the lower limbs.

### BACKGROUND OF THE INVENTION

As is well known, in vascular complaints that affect the lower limbs, such as diabetic foot, which according to the International Consensus on Diabetic Foot is an infection, ulceration or destruction of deep tissues related to neurological alterations and different degrees of peripheral vascular disease in the lower limbs that affect patients with Diabetes Mellitus that has not been, or is not being, correctly treated, the application of heat-based treatments, specifically treatment by means of radio-frequency waves, is known, since they produce biological effects due to the direct heating of the tissues that are located at a greater depth.

In this sense, different types and models of devices for applying these heat treatments to different areas of the body are known on the market. Said devices are usually made up of heat-generating equipment and one or more means of transmitting and applying said energy, normally electrodes, which are positioned on the area of the body to be treated, being connected to the device by means of the corresponding cable.

These treatments, based on radio-frequency, are based on the application of small electric currents and on frequencies and power determined by specific wave shapes, through electrodes, which can be two types:
- Monopolar, in which the radio-frequency conducted is applied by means of two electrodes: one return electrode in a fixed position, incorporated in a passive plate that is located under the area being treated, and another active plate that is located on the other side of the area being treated and which can be in a fixed position, incorporated in an active or mobile plate, incorporated in an element that moves.
- Bipolar, which include the active and passive part of the radio-frequency circuit in a single element. In turn, this bipolar electrode can be in a fixed position (bipolar plate) or a mobile position.

In any case, at least when we are dealing with applying the treatment to the lower limbs, it is the therapist who must hold the plates with the electrodes to the areas of the affected foot, and thus they must hold one or two plates during the entire session and make sure that they are kept in contact with the patient's skin and in the correct position in order for there to be a proper interaction and for the effect of the radio-frequency waves to be optimum.

Therefore, this type of treatment, which, on the one hand is very effective, is usually only applied in clinics, doctor's offices or establishments that are specialized and private and is not usually applied in public hospitals or health institutions, due to the fact that the need for a therapist to be continuously monitoring each patient receiving treatment incurs costs for maintaining medical personnel that run too high.

Thus, the object of the present invention is to provide the market, and particularly all health institutions and establishments, both public and private, with a new type of foot accessory for applying said type of treatment using radio-frequency waves on patients with vascular complaints who are in need of such treatment, without the need for a therapist to be constantly monitoring the patient, and which, after the placement of the device, allows for the autonomous fastening of the electrodes , making it only necessary for the patient to be monitored every once in a while to ensure the correct application of the treatment, or even no monitoring at all, until the end of each treatment session.

On the other hand, and as a reference to the current state of the art, it is worth mentioning that the existence of any other foot device for treating vascular complaints in the lower limbs is unknown, at least by the applicant, and furthermore, so is any other similar application that has technical, structural and constituent characteristics that are equal or similar to those specifically advocated herein, as claimed.

### DESCRIPTION OF THE INVENTION

The foot device for treating vascular complaints in the lower limbs proposed by the invention is configured as a novelty within its field of application, given that in light of its implementation, and to the highest degree, the previously mentioned objectives are satisfactorily reached, the characterizing details which make the invention possible and which distinguish it, being conveniently established in the final claims that accompany the present description.

As was previously mentioned, what the invention proposes is a device intended to facilitate the application of radio-frequency treatments in the lower limbs of patients who suffer from vascular complaints in said limbs, such as for example diabetic foot, for which the device has a specific structural configuration that provides an autonomous fastening of the electrodes to the skin of the patient's foot, with complete interaction of the electrodes with the same, all of which is able to be used in a quick, practical and simple way, and without the need for the continuous monitoring of the application of the treatment by a therapist or professional applying the treatment.

The foot device further includes electroporation means that allow for a significant increase in the electrical conductivity and the permeability of the plasma cell membrane for introducing molecules of active medication in the tissues of the foot of the patient/user to heal and repair ulcers and cutaneous lesions resulting from the circulatory and vascular complaint of the diabetic foot.

Said substances can be hydrating medication, such as hyaluronic acid applied topically, antibiotic or healing substances, the intrinsic vascular effect complementing the radio-frequency emitted by the generating device.

To this end, the device is essentially made up of the following elements:
- a support structure, made from any suitable rigid material, such as rigid plastic, for example, which has an L-shaped configuration, sized to accommodate and act as a support to the rear end part of the leg and the sole of the patient's foot,
- and an inner casing or covering made of a soft material, which covers at least the inner face of the support structure, acting as a base that is adjustable to the morphology of the patient's leg and foot, into which the electrodes for applying radio-frequency waves are integrated, for which they are conveniently provided with cables for the connection thereof to the equipment that provides the necessary energy for them, and from which the power and wavelength is controlled.

And furthermore, for electroporation means that increase the electrical conductivity and the permeability of the plasma cell membrane for introducing molecules of active medication, the device incorporates an additional circuit that is able to be connected to the electrodes which, combined with the use of a conductive cream, provides said electroporation capability.

Logically, said electrodes, preferably embedded in a plate, are integrated in the soft casing, such that they are flush with the surface of the same that will be in contact with the patient's skin, when the patient puts on the conveniently fastened device, and thus it is provided with corresponding securing means, preferably adjustable straps with buckle closures, velcro, or other types.

Preferably, the device incorporates two electrode plates, one with the passive electrode integrated in the soft casing in the area that covers the rear lower part of the leg, and another with the active electrode located in the soft casing in the area that covers the sole of the foot.

Furthermore, in the preferred embodiment, the existence of an adapter in the form of a gel pack is envisaged on the active electrode that is situated in the area that covers the sole of the foot, so as to provide an integral contact with the entire area of the sole of the foot, given that the same is not normally flat and may have a high degree of unevenness between one area and another, especially in the central arch of the foot.

Also optionally, one or both electrode plates are provided with temperature sensors.

Moreover, in an alternative variant embodiment, the device incorporates one or more LEDs of one or more colors integrated in the electrode plate or next to the same, so as to provide complementary treatments of phototherapy in cases where patients require said treatment.

In any case, the device is additionally provided with a hygienic protective cover, preferably made of single-use disposable plastic material, which is incorporated covering at least the inner area of the same, meaning the area that has the electrode plates integrated in the soft casing, given that it is the area that comes into contact with the user's skin, able to have an elongated rectangular configuration and made of a single layer, to be simply placed on said area, or which could consist of a type of sack in which the complete structure is inserted, although as an alternative, another possibility could be that said cover is configured in the form of a sock so that it is the patient who puts it on.

It is envisaged that the boot is to be used with an individual plastic cover which makes it so each patient is individual and hygienic, since in this way the energy-emitting electrodes do not come into contact with the patient's skin, but rather the electrodes only touch the protective cover.

Furthermore, the electrical conduction, as was previously mentioned, is carried out using a conductive cream that transfers the energy from the boot through a pad full of gel incorporated by the boot, and by crossing the cover it is conducted, thanks to said cream, to the body of the patient.

Unlike other patents, the passive plate receiving the energy is not in contact with the skin, but rather remains outside the hygienic plastic cover.

Lastly, it is worth mentioning that, optionally, the support structure of the device of the invention is made up of two pieces articulated to one another, one which covers the rear lower area of the leg and the other which covers the sole of the foot, both parts being joined by an articulated joint that allows for a certain degree of mobility between the two so as to vary the angle formed by the same and thereby allow for an adjustment of the same to people who, for various reasons, cannot correctly place their foot in a right angle with respect to their leg, such that it is the structure that adjusts to the angle of the foot of the patient, thereby ensuring a proper contact for a perfect interaction of the electrodes with the patient's skin.

In light of the foregoing, it is clear that the previously described foot device for treating vascular complaints in the lower limbs is an innovative structure with constituent and structural characteristics, heretofore unknown, for its intended purpose, reasons which, taken together with its usefulness, provide it with sufficient grounds for obtaining the requested exclusivity privilege.

### DESCRIPTION OF THE DRAWINGS

As a complement to the present description, and for the purpose of helping to make the characteristics of the invention more readily understandable, said description is accompanied by a set of drawings constituting an integral part of the same, which by way of illustration and not limitation represent the following:
Figure number 1 shows a side perspective view of an example of the foot device for treating vascular complaints in the lower limbs, object of the invention, in which one can see the main parts and elements of which it is comprised, as well as the configuration and arrangement of the same.

### PREFERRED EMBODIMENT OF THE INVENTION

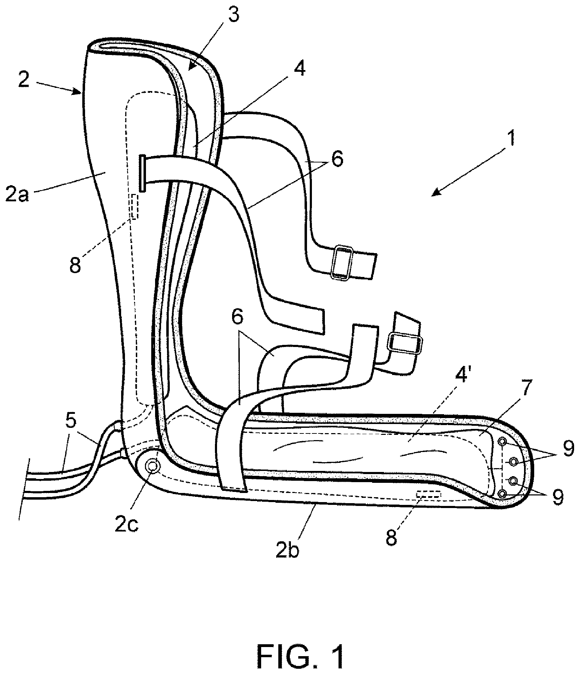

In light of figure 1, the only figure, and according to the numbering used, one can see a non-limiting exemplary embodiment of the foot device for treating vascular complaints in the lower limbs, herein recommended, which comprises the parts and elements that are indicated and described in detail below.

As can be seen in said figure, the foot device (1) in question is essentially configured from a support structure (2), with an L-shaped configuration, which has an inner covering (3) made of soft material, into which the electrode plates (4, 4') for applying radio-frequency waves are integrated, being connected by means of the corresponding cables (5) to a generator (not shown), determining a base adjustable to the morphology of the inner part of the leg and foot of the patient so as to establish an optimum contact surface between said electrode plates (4, 4') and the surface of the skin in said areas of the lower extremity.

Furthermore, said electrode plates (4, 4') are connected to an additional circuit of the generator which, combined with the use of a conductive cream, provides the device with the electroporation capability to introduce molecules of active medication by significantly increasing the electric conductivity of the applied electric field and the permeability of the plasma cell membrane.

The support structure (2) is a body made of rigid material, such as plastic, and preferably comprises a vertical branch (2a), sized to accommodate and act as a support to the area of the rear end part of the patient's leg, and a horizontal branch (2b) sized to accommodate the sole of the patient's foot.

Optionally, the two branches (2a, 2b) comprised by the body that makes up the support structure (2) are two independent pieces coupled to one another by means of an articulated joint (2c), which allows for a certain degree of mobility between the same to vary the angle formed between the two and to be able to adjust it to the different needs of patients with mobility problems.

As for the inner covering (3), it is made of soft material, which for example is foam, and covers at least the inner face of the two branches (2a, 2b) of the support structure (2), incorporating the electrode plates (4, 4') embedded in the same and flush with its surface, the respective cables (5) of which are inserted through holes provided in said inner covering (3) and in the support structure (2), to emerge from the outer or rear part of the assembly, so as to avoid any inconvenience to the user.

Preferably, the device (1) incorporates two electrode plates, one with a passive electrode (4) integrated in the inner covering (3) of the vertical branch (2a) which covers the rear lower part of the patient's foot, making contact with said part and another plate with an active electrode (4') integrated in the inner covering (3) of the horizontal branch (2b) of the structure which covers the sole of the patient's foot, making contact with the surface of the same when the patient puts on the device (1), which, in any case, has securing means (6) for autonomously keeping the same attached to the limb and for guaranteeing proper contact of both electrode plates (4, 4') with the skin of the patient.

Preferably, said securing means (6) consist of pairs of straps with an adjustable fastening, to be able to adjust to different sizes of the limbs of different patients, one on the vertical branch (2a) and the other on the horizontal branch (2b) of the support structure (2), in both cases with easy-to-open fasteners, such as buckles or velcro.

Optionally, the existence of an adapter (7), preferably a gel pack, is envisaged on the active electrode plate (4') which is embedded in the covering (3) of the horizontal branch (2b) of the structure, so as to optimize the contact with the entire surface of the sole of the foot, which is generally not flat, but rather quite uneven.

Optionally, one or both electrode plates (4, 4') also have a temperature sensor (8) and one or more LEDs (9) for phototherapy treatment.

In any case, and although not represented in the figure, the device (1) of the invention envisages the existence of a hygienic protective cover, preferably made of a single-use disposable plastic material, which is incorporated covering at least the inner area of the same where the electrode plates (4, 4') are placed, having an elongated rectangular form, and made of a single layer, which is arranged on said inner area, or in the form of a sack that houses the entire structure (2) of the device (1), or in the form of a sock for the patients to put on before putting on the device (1).

Having sufficiently described the nature of the present invention, as well as the ways of implementing it, it is not considered necessary to extend its explanation for any expert in the state of the art to understand its scope and the advantages which derive from it, specifying that, within its essential nature, it can be carried out in other embodiments that differ in detail from the one provided by way of example, and which are also covered by the requested protection, provided that they do not alter, change or modify its fundamental principle.

## Claims

1. A FOOT DEVICE FOR TREATING VASCULAR COMPLAINTS IN THE LOWER LIMBS, in particular radio-frequency treatments by means of electrodes (4,4') applied in contact with the skin of the affected area and connected by means of cables (5) to a generator and control device, **characterized in that** it comprises a support structure (2), with an L-shaped configuration, which has an inner covering (3) made from soft material, into which the electrode plates (4, 4') for applying radio-frequency waves are integrated, determining a base that is adjustable to the morphology of the lower part of the leg and foot of the patient, and establishing the contact surface between said electrode plates (4, 4') and the surface of the skin in said areas of the lower extremity; **in that** said support structure (2) has securing means (6) for autonomously keeping the same attached to the limb and keeping the electrode plates (4, 4') in contact with the patient's skin; **in that** the electrode plates (4, 4') connect to an additional circuit of the generator which, combined with a conductive cream, significantly increases the electrical conductivity of the applied electric field and the permeability of the plasma cell membrane, providing it with electroporation capability for introducing molecules of active medication.

2. THE FOOT DEVICE FOR TREATING VASCULAR COMPLAINTS IN THE LOWER LIMBS, according to claim 1, **characterized in that** the support structure (2) is a body made of rigid material.

3. THE FOOT DEVICE FOR TREATING VASCULAR COMPLAINTS IN THE LOWER LIMBS, according to claim 2, **characterized in that** the support structure (2) comprises a vertical branch (2a), sized to accommodate and act as a support to the area of the rear end part of the patient's leg, and a horizontal branch (2b), sized to accommodate the sole of the patient's foot.

4. THE FOOT DEVICE FOR TREATING VASCULAR COMPLAINTS IN THE LOWER LIMBS, according to claim 3, **characterized in that** the two branches (2a, 2b) comprised by the body that makes up the support structure (2) are two independent pieces coupled to one another by means of an articulated joint (2c) that allows for mobility between the two to vary the angle they form with respect to one another.

5. THE FOOT DEVICE FOR TREATING VASCULAR COMPLAINTS IN THE LOWER LIMBS, according to any of claims 3 or 4, **characterized in that** the inner covering (3) made of soft material covers at least the inner face of the two branches (2a, 2b) of the support structure (2), incorporating the electrode plates (4, 4'), embedded in the same and flush with the surface, the respective cables (5) of which are inserted through the holes provided in said inner covering (3) and in the support structure (2), to emerge through the outer or rear part of the assembly.

6. THE FOOT DEVICE FOR TREATING VASCULAR COMPLAINTS IN THE LOWER LIMBS, according to any of claims 1 to 5, **characterized in that** the device (1) incorporates two electrode plates, one with a passive electrode (4) and the other with an active electrode (4').

7. THE FOOT DEVICE FOR TREATING VASCULAR COMPLAINTS IN THE LOWER LIMBS, according to any of claims 3 to 5, **characterized in that** it has a passive electrode plate (4) integrated in the inner covering (3) of the vertical branch (2a) that accommodates the rear lower part of the leg of the patient, and another plate with an active electrode (4') integrated in the inner covering (3) of the horizontal branch (2b) of the structure that accommodates the sole of the patient's foot.

8. THE FOOT DEVICE FOR TREATING VASCULAR COMPLAINTS IN THE LOWER LIMBS, according to any one of claims 3 to 7, **characterized in that** the existence of an adapter (7) in the form of a gel pack is envisaged on the electrode plate that is located embedded in the covering (3) of the horizontal branch (2b) in order to optimize the contact with the entire surface of the sole of the foot.

9. THE FOOT DEVICE FOR TREATING VASCULAR COMPLAINTS IN THE LOWER LIMBS, according to any of claims 1 to 8, **characterized in that** the securing means (6) consist of adjustable straps, so as to be able to adjust to different sizes of the limbs of different patients, with easy-to-open fasteners, such as buckles or velcro.

10. THE FOOT DEVICE FOR TREATING VASCULAR COMPLAINTS IN THE LOWER LIMBS, according to any of claims 1 to 9, **characterized in that** it has a temperature sensor (8).

11. THE FOOT DEVICE FOR TREATING VASCULAR COMPLAINTS IN THE LOWER LIMBS, according to any of claims 1 to 10, **characterized in that** it has one or more LEDs (9).

12. THE FOOT DEVICE FOR TREATING VASCULAR COMPLAINTS IN THE LOWER LIMBS, according to any of claims 1 to 10, **characterized in that** it envisages the existence of a hygienic protective cover made of single-use disposable plastic material that is incorporated covering at least the area of the same where the electrode plates (4, 4') go.

## Amended claims

### Amended claims under Art. 19.1 PCT

1. A foot device for treating vascular complaints in the lower limbs, in particular radio-frequency treatments by means of electrodes (4,4') applied in contact with the skin of the affected area and connected by means of cables (5) to a generator and control device, **characterized in that** it comprises:
- a support structure (2), made of rigid material, with an L-shaped configuration, comprising a vertical branch (2a), frontally opened, sized to accommodate and act as a support to the area of the rear end part of the patient's leg, and a horizontal branch (2b), open at the top, sized to accommodate the sole of the patient's foot,
- an inner covering (3) made from soft material, into which the electrode plates (4, 4') for applying radio-frequency waves are integrated, determining a base that is adjustable to the morphology of the lower part of the leg and foot of the patient, and establishing the contact surface between said electrode plates (4, 4') and the surface of the skin in said areas of the lower extremity; and
- a securing means (6) of the support structure (2) for autonomously keeping the same attached to the limb of the patient and keeping the electrode plates (4, 4') in contact with the patient's skin.

2. Foot device according to claim 1, **characterized in that** the two branches (2a, 2b) comprised by the body that makes up the support structure (2) are two independent pieces coupled to one another by means of an articulated joint (2c) that allows for mobility between the two to vary the angle they form with respect to one another.

3. Foot device according to any of claims 1 and 2, **characterized in that** the inner covering (3) made of soft material covers at least the inner face of the two branches (2a, 2b) of the support structure (2), incorporating the electrode plates (4, 4'), embedded in the same and flush with the surface, the respective cables (5) of which are inserted through the holes provided in said inner covering (3) and in the support structure (2), to emerge through the outer or rear part of the assembly.

4. Foot device according to any of claims 1 to 3, **characterized in that** the device (1) incorporates two electrode plates, one with a passive electrode (4) and the other with an active electrode (4').

5. Foot device according to claim 4, **characterized in that** it has a passive electrode plate (4) integrated in the inner covering (3) of the vertical branch (2a) that accommodates the rear lower part of the leg of the patient, and another plate with an active electrode (4') integrated in the inner covering (3) of the horizontal branch (2b) of the structure that accommodates the sole of the patient's foot.

6. Foot device according to claim 5, **characterized in that** the existence of an adapter (7) in the form of a gel pack is envisaged on the electrode plate that is located embedded in the covering (3) of the horizontal branch (2b) in order to optimize the contact with the entire surface of the sole of the foot.

7. Foot device according to any of claims 1 to 6, **characterized in that** the securing means (6) consist of adjustable straps, so as to be able to adjust to different sizes of the limbs of different patients, with easy-to-open fasteners, such as buckles or velcro.

8. Foot device according to any of claims 1 to 7, **characterized in that** it has a temperature sensor (8).

9. Foot device according to any of claims 1 to 8, **characterized in that** it has one or more LEDs (9).

10. Foot device according to any of claims 1 to 9, **characterized in that** it is provided a hygienic protective cover made of single-use disposable plastic material that is incorporated covering at least the area of the same where the electrode plates (4, 4') are.
